Europäisches Patentamt

European Patent Office

Office européen des brevets

(11) Publication number: **0 239 116 B1**

# EUROPEAN PATENT SPECIFICATION

(45) Date of publication of patent specification: **26.05.93** (51) Int. Cl.⁵: **G01N  33/20**

(21) Application number: **87104527.4**

(22) Date of filing: **26.03.87**

(54) Method for determining deoxidant concentration in molten metal.

(30) Priority: **27.03.86 US 844557**

(43) Date of publication of application:
**30.09.87 Bulletin  87/40**

(45) Publication of the grant of the patent:
**26.05.93 Bulletin  93/21**

(84) Designated Contracting States:
**BE DE FR IT**

(56) References cited:
**BE– A– 684 624**
**DE– A– 3 437 041**
**FR– A– 2 248 506**
**US– A– 2 253 574**

(73) Proprietor: **UNION CARBIDE CORPORATION**
**39 Old Ridgebury Road**
**Danbury Connecticut 06817(US)**

(72) Inventor: **Riley, Michael Francis**
**21 Westwood Drive**
**Danbury, Ct.06811(US)**

(74) Representative: **Schwan, Gerhard, Dipl.– Ing.**
**Elfenstrasse 32**
**W– 8000 München 83 (DE)**

Rank Xerox (UK) Business Services
(3. 10/3.6/3.3. 1)

## Description

### Technical Field

This invention relates generally to metallurgy, such as steelmaking, and, more particularly, is an improvement to that portion of the process comprising the chemical analysis of the metal melt to ensure proper melt chemistry prior to the pouring of the molten metal into molds to form ingots or final product castings.

### Background Art

Many metals, such as steels, are refined by the injection of air, enriched air or oxygen into the metal melt to oxidize components in the melt. One example of this is the decarburization of a steel melt wherein an oxygen-containing gas or pure oxygen is injected into the melt to react with carbon in the melt to form carbon monoxide which then bubbles out of the melt. One of the most successful commercial decarburization processes is the Argon-Oxygen Decarburization (AOD) Process wherein oxygen and an inert gas, such as argon, are injected into a steel melt from below the melt surface. It is imperative for the production of metal of good integrity that, after the oxygen refining step(s) is completed and the melt is ready for pouring into molds, the oxygen concentration of the melt be relatively low.

In order to ensure that the oxygen concentration of the melt is acceptable prior to pouring the molten metal into molds, metallurgists often add a deoxidant to the melt after the melt has been refined. The deoxidant combines with dissolved oxygen in the melt to form an oxide which then floats up into the slag and out of the melt. Since the deoxidant has a greater affinity for oxygen than does the metal which has been refined, the deoxidant also serves to keep the oxygen from oxidizing the refined melt and thus serves to increase the yield of the metal making process.

Additives which may be employed as deoxidants include aluminum, silicon, titanium vanadium, calcium, zirconium, and the rare earth metals. In addition to their use as a deoxidant each of these elements serves other valuable functions.

Aluminum forms an effective fine dispersion with nitrogen or oxygen that restricts austenite grain growth and also forms, by relatively low-temperature diffusion of nitrogen, an exceedingly effective surface-hardened layer, i.e., nitriding.

Silicon, in low-carbon compositions, aids in the production of desired crystal orientations and raises electrical sensitivity, contributes oxidation resistance in certain heat-resisting compositions, moderately increases hardenability in steels carrying other non-graphitizing elements, and increases strength in quenched and tempered steels and pearlitic steels wherein plasticity is not sought.

Titanium withdraws carbon from solution and reduces martensitic hardness and hardenability in medium-chromium steels, especially during welding, prevents austenite formation in high chromium steels, withdraws carbon from solution at elevated temperature in austenitic stainless steels and thereby prevents intergranular deterioration through chromium-carbide formation at grain boundaries and attendant local chromium depletion, and serves as a precipitation hardening agent in austenitic high temperature alloys containing nickel.

Vanadium elevates coarsening temperature of austenite with advantages of fine grain, increases hardenability, and increases resistance to softening in tempering, amounting, in moderate concentration, to marked secondary hardness.

Calcium increases the productivity in machining operations on carbon and low-alloy steels by converting abrasive aluminum oxide inclusions to less abrasive calcium aluminates. Calcium, zirconium, and the rare earth metals all act to control the shape of sulfide inclusions, resulting in improved mechanical properties.

It is important that the concentration of the deoxidant in the melt be accurately known to the steelmaker, in order to effectively carry out deoxidation and/or other functions described above, prior to pouring the molten metal into molds. If the melt contains deoxidant significantly below or in excess of a predetermined target amount, the metal may be out of its specification and may suffer quality defects.

Generally the deoxidant concentration of a metal melt is determined by taking a sample of the melt and running a chemical analysis of the sample. If the chemical analysis shows that the deoxidant concentration is within the predetermined specification range, the melt is then poured into the molds; if not, then adjustments to the melt are made, and another sample is taken.

This chemical analysis method has been generally satisfactory and accurate but can be time consuming. This is detrimental for two reasons. First valuable production time is lost since further processing of the melt is not possible until the chemical analysis has been made. Second, during the time it takes to make the chemical analysis, the melt is constantly losing heat. This requires that the melt be processed at a higher temperature to compensate for these losses, adding to processing costs. Moreover, should the melt temperature fall below the requisite tap temperature, the melt would have to undergo a costly reheating step to raise its temperature into the proper temperature range. A melt that is below the proper temperature range will not pour correctly and/or will not solidify in the molds properly resulting in poor quality product or unusable scrap.

Those skilled in the art have addressed this problem by devising a new way to determine the deoxidant level of a metal melt. This method takes advantage of the deoxidizing capacity of the additive to determine its concentration. In this method an oxygen probe is used to determine the oxygen activity of the steel melt. The oxygen activity is then used to determine the deoxidant concentration by use of the thermodynamic relationship of oxygen with the particular deoxidant being employed. Since the probe can determine the oxygen activity very quickly, the deoxidant concentration of the melt can be determined much more rapidly than with the chemical analysis method.

Unfortunately the conventional oxygen probe method has demonstrated disappointing accuracy, and therefore has had limited usefulness, when the molten metal bath contains a complex slag or where there are complex deoxidation products in the melt. Complex slags and complex deoxidation products are characterized by a solution of deoxidant oxides and metal oxides.

It is therefore an object of this invention to provide a method for rapidly and accurately determining the deoxidant concentration in a molten metal bath containing a complex slag and/or complex deoxidation products by use of an oxygen probe.

## Summary of the Invention

The above and other objects which will become apparent to one skilled in the art upon a reading of this disclosure are attained by the method of this invention which is:

a method for determining the deoxidant concentration in a molten metal bath by employing the activity of oxygen in the bath determined by use of an oxygen probe, comprising the steps of:

(a) making a deoxidant addition to a molten metal bath containing dissolved oxygen and metal oxide(s);

(b) reacting at least some of the deoxidant with dissolved oxygen in the bath to form deoxidant oxide(s) which in turn form a solution with said metal oxides(s); and

(c) stirring the molten metal bath by the subsurface injection of gas into the bath for a period of time, at least equal to 9t, prior to use of the oxygen probe, where

$t = 800\ E^{-0.4}$, and

$E = [7250 + 9850 \ln (1 + 0.676\ h)]\ V/W$

wherein t is in seconds, h is the height in meters of the bath surface above the stirring gas injection point, V is the stirring gas flow rate in standard cubic meters per minute, and W is the weight of the bath in metric tons

$$(E = [226 + 307 \ln (1 + 0.206\ h)]\ V/W,$$

wherein t is in seconds, h is the height in feet of the bath surface above the stirring gas injection point, V is the stirring gas flow rate in standard cubic feet per minute, and W is the weight of the bath in short tons).

As used herein, the term "bath" means the contents inside a metal refining vessel, and comprising a melt, which comprises molten metal and material dissolved in the molten metal, and a slag, which comprises material not dissolved in the molten metal.

As used herein, the term "activity" means the ratio of the fugacity of a substance in a certain state to the fugacity of that substance in a given standard state.

As used herein, the terms "argon oxygen decarburization process" or "AOD process" mean a process for refining molten metals and alloys contained in a refining vessel provided with at least one submerged tuyere comprising:

(a) injecting into the melt through said tuyere(s) an oxygen-containing gas containing up to 90 percent of a dilution gas, wherein said dilution gas may function to reduce the partial pressure of the carbon monoxide in the gas bubbles formed during decarburization of the melt, alter the feed rate of oxygen to the melt without substantially altering the total injected gas flow rate, an/or serve as a protective fluid, and thereafter

(b) injecting a sparging gas into the melt through said tuyere(s) said sparging gas functioning to remove impurities from the melt by degassing, deoxidation, volatilization or by floatation of said impurities with subsequent entrapment or reaction with the slag. Useful dilution gases include argon, helium, hydrogen, nitrogen, steam or hydrocarbon. Useful sparging gases include argon, helium, hydrogen, nitrogen, carbon monoxide, carbon dioxide, steam and hydrocarbons. Liquid hydrocarbons and carbon dioxide may also be employed as protective fluids. Argon and nitrogen are the preferred dilution and sparging gas. Argon, nitrogen and carbon dioxide are the preferred protective fluids.

## Brief Description of the Drawing

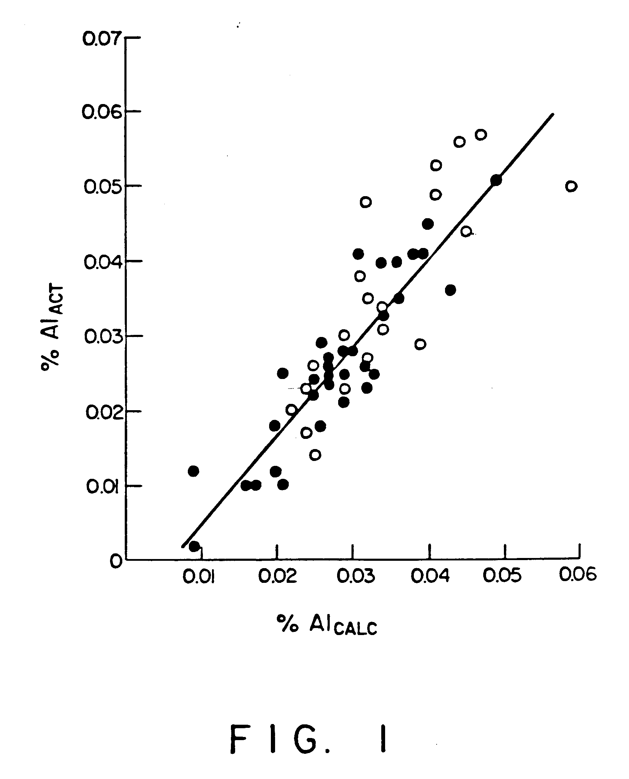

Figure 1 is a graphical representation of the results of a number of trials of the process of this invention as compared to the actual measured results. For comparative purposes Figure 1 also shows the results of a number of trials wherein a conventional oxygen probe technique was used to determine deoxidant concentration, and a comparison of these results to the actual measure results.

Detailed Description

The present invention is a method for rapidly determining the deoxidant concentration of a molten metal bath containing a complex slag and/or complex deoxidation product(s) in the melt.

The method of this invention is most advantageously employed after molten metal has been refined and prior to its being poured into molds. Among the molten metals which can be advantageously analyzed by the method of this invention one can name steel, such as stainless steel, structural steel, carbon and low alloy steel and tool steel, as well as nickel−based alloys, copper and copper−based metals and any other metal which is refined with slag or deoxidized by elements forming a complex oxide. When the metal is steel, a preferred refining method is the AOD process.

In the method of this invention, deoxidant is added to a molten metal bath containing dissolved oxygen and metal oxide(s). Any effective deoxidant which can form complex oxides may be used, such as aluminum, silicon, titanium, vanadium, calcium, zirconium or a rare earth metal. A single deoxidant may be employed or two or more different deoxidants may be used. The preferred deoxidant is aluminum.

The quantity of deoxidant added to the bath will vary and will depend upon such factors as the size of the melt, the melt specification, the level of dissolved oxygen in the melt, the presence of weaker oxides in the slag, and whether or not oxygen will be added to the bath.

Generally the deoxidant is added as a batch addition, but it may be also blown into the melt using techniques known to those skilled in the art, or added to the melt as a continuously fed wire.

The deoxidant reacts with oxygen which is dissolved in the bath to form deoxidant oxide(s). For example when aluminum is employed as the deoxidant, aluminum oxide ($Al_2O_3$) is a resulting deoxidant oxide. The deoxidant oxide may then float up into the slag and form a complex slag by forming a solution with metal oxides such as iron or chromium oxides and with basic slag components such as calcium or magnesium oxides which are often present in such slags. The deoxidant oxide may also form a solution with metal oxides within the melt itself.

After the deoxidant is added to the bath, and prior to the use of the oxygen probe to determine the oxygen activity, the bath is stirred, by the subsurface injection of gas, for a requisite minimum stirring time.

As is known in the art, each metallurgical vessel has a characteristic mixing time which is defined by

$$t = 800E^{-0.4}$$

where t is the characteristic mixing time in seconds and

$$E = [7250 + 9850 \ln (1 + 0.676 h)] V/W$$

wherein t is in seconds, h is the height in meters of the bath surface above the stirring gas injection point, V is the stirring gas flow rate in standard cubic meters per minute, and W is the weight of the bath in metric tons

$$(E = [226 + 307 \ln(1 + 0.206h)]V/W$$

where E is the specific stirring energy in watts per ton, h is the height in feet of the bath surface above the stirring gas injection point, V is the stirring gas flow rate in standard cubic feet per minute, and W is the weight of the bath in short tons). For a typical AOD vessel the characteristic mixing time t is generally about 20 seconds. For a typical powder injection system the characteristic mixing time t is generally about 26 seconds.

Applicant has found that, in the case where the molten metal bath contains a complex slag and/or where there are complex deoxidation products in the melt, the accuracy of the determination of the deoxidant concentration by the use of an oxygen probe is markedly increased if the deoxidant addition is followed by stirring for at least 9 times the characteristic mixing time t for the vessel, prior to the use of the oxygen probe. In a typical AOD vessel, for example, this important minimum stirring time would be 9 times 20 seconds, or about 3 minutes.

The stirring may be carried out by any effective subsurface gas injection method employing any effective gas. One preferred way of injecting the stirring gas into the melt is through one or more tuyeres located through the side or the bottom of the metallurgical vessel. Preferred stirring gases include argon and nitrogen.

After the bath is stirred for the requisite time the oxygen activity of the molten metal is determined by use of an oxygen probe. Oxygen probes and their use to determine oxygen activity in a melt are well known in the art and several are commercially available such as Electro−Nite Celox®, Leeds and Northrup Temp − o − Tip, Leco Oxygen Probe, and Minco Minex.

In general, an oxygen probe comprises a thermocouple for measuring the temperature of the molten metal and an electrical circuit which com − pares the oxygen activity of the molten metal bath with that of a known standard source. The source generally is a mixture of chromium metal and chromium oxide powders. Placing the standard source and the bath in the same electrical circuit generates a voltage which can be related to the bath oxygen activity through the known thermody − namics of the reaction:

$$2Cr + 3O = Cr_2O_3$$

Once the oxygen activity has been measured by the oxygen probe, the level of deoxidant in the metal can be calculated in a manner known to the art from the known thermodynamics of the reaction

$$xD + yO = D_xO_y$$

where D is the deoxidizing element and x and y are stoichiometric coefficients. When the slag or deoxidation product is pure $D_xO_y$, the level of D can be calculated directly. Under complex slags or deoxidation products, i.e., those containing a solu − tion of the deoxidant oxide and other metal oxides, the activity of the deoxidant oxide must be deter − mined before the level of D can be found. One way to do this is to chemically analyze a solution of deoxidant oxide(s) and metal oxide(s) to determine the concentration of the deoxidant oxide(s) which, through a known correspondence, enables one to determine the activity of the deoxidant oxide(s) in the bath. Generally, however, it is most convenient to assume a reference activity of the deoxidant oxide and calculate an associated reference level of deoxidant. This reference level can then be correlated empirically to the actual value, as de − termined by chemical analysis, over several test heats. The slope of this correlation is related to the deviation of the actual deoxidant oxide activity from the reference value and, at low oxygen values, to the error signal generated by the electrical con − ductivity of the probe. Alternatively, the correlation can be calculated from relating a slag analysis to known slag activity data and from known oxygen probe electrical conductivity data. In practice, however, the empirical method is easier and therefore preferable, although the empirical data can be shown to be consistent with the calculated data. Regardless of the method of correlation, however, that correlation holds for any given prac − tice producing similar alloys under similar slag composition. Conversely, when making alloys where the deoxidant has significantly different thermodynamic properties or when processing under significantly different slag compositions, a different correlation may be required.

The method of this invention was employed to determine the deoxidant concentration of 26 dif − ferent heats of structural steel. Each steel heat had been refined by the AOD process in a 30 metric ton (33 short ton) AOD vessel and was deoxidized by the addition of aluminum. Following the alu − minum addition, each heat was stirred for at least three minutes by the subsurface injection of 57 standard $m^3$ (2000 SCF) of argon, and a Electro − Nite Celox® Cx12 CLL 10 48 oxygen probe was used to measure the oxygen activity from which the deoxidant concentration was calculated. Si − multaneous with the oxygen probe reading a sam − ple of the melt was taken and this sample was later chemically analyzed for the deoxidant concentra − tion. A total of 34 such measurements were taken. The deoxidant concentration calculated by the method of this invention was compared to the corresponding deoxidant concentration determined by chemical analysis and each comparison is plotted on Figure 1 as a solid dot.

For comparative purposes the procedure de − scribed above was repeated except that the bath was stirred for less than three minutes following the deoxidant addition prior to the oxygen probe mea − surement and sampling. Twenty such measure − ments were taken and each comparison is plotted on Figure 1 as an open dot.

The line passing through the 54 data points in Figure 1 is a best fit regression correlation for the data. The scatter around this correlation gives a 95 percent confidence interval of ± 0.015 percent aluminum for trials preceded by less than three minutes of stirring while the scatter around this correlation gives a 95 percent confidence interval of ± 0.009 percent aluminum for the trials carried out in accord with the method of this invention. As can be seen the method of this invention enabled an increase in accuracy of 40 percent.

Furthermore the method of this invention en − ables one to determine the concentration of deox − idant in molten metal with an accuracy comparable to that possible with chemical analysis while sig − nificantly reducing the time necessary to make the determination. Generally the time savings per heat will be 10 minutes or more.

Now by the use of applicant's invention one can rapidly and accurately determine the deoxidant concentration in molten metal despite the presence of a complex slag and/or complex deoxidation products.

## Claims

1. A method for determining the deoxidant concentration in a molten metal bath by employing the activity of oxygen in the bath determined by use of an oxygen probe, comprising the steps of:

   (a) making a deoxidant addition to a molten metal bath containing dissolved oxygen and metal oxide(s);

   (b) reacting at least some of the deoxidant with dissolved oxygen in the bath to form deoxidant oxide(s) which in turn form a solution with said metal oxide(s); and

   (c) stirring the molten metal bath by the subsurface injection of gas into the bath for a period of time, at least equal to 9t, prior to use of the oxygen probe, where

   $t = 800\ E^{-0.4}$, and
   $E = [7250 + 9850\ \ln (1 + 0.676\ h)]\ V/W$

   wherein t is in seconds, h is the height in meters of the bath surface above the stirring gas injection point, V is the stirring gas flow rate in standard cubic meters per minute, and W is the weight of the bath in metric tons

   $(E = [226 + 307\ \ln (1 + 0.206\ h)]\ V/W$,

   wherein t is in seconds, h is the height in feet of the bath surface above the stirring gas injection point, V is the stirring gas flow rate in standard cubic feet per minute, and W is the weight of bath in short tons).

2. The method of claim 1 wherein the deoxidant is aluminum.

3. The method of claim 1 wherein the deoxidant is silicon.

4. The method of claim 1 wherein the deoxidant is both aluminum and silicon.

5. The method of claim 1 wherein the molten metal is steel.

6. The method of claim 1 wherein the molten metal is a nickel-based alloy.

7. The method of claim 1 wherein the stirring gas is argon.

8. The method of claim 1 wherein the stirring gas is nitrogen.

9. The method of claim 1 wherein the deoxidant is added as a batch addition.

10. The method of claim 1 wherein the deoxidant addition is made as a powder blown into the melt with gas.

11. The method of claim 1 wherein the deoxidant addition is made as a wire which is continuously fed into the melt.

12. The method of claim 1 wherein the deoxidant is from the group comprising titanium, vanadium, calcium, zirconium and the rare earth metals.

13. The method of claim 5 wherein the melt is refined by the argon oxygen decarburization process (AOD method).

14. The method of claim 13 wherein the stirring time is at least 3 minutes.

## Patentansprüche

1. Verfahren zum Feststellen der Desoxidationsmittelkonzentration in einem Metallschmelzbad unter Verwendung der durch Einsatz einer Sauerstoffsonde bestimmten Sauerstoffaktivität in dem Bad, bei dem:

   (a) einem Metallschmelzbad, das gelösten Sauerstoff und Metalloxid (e) enthält, Desoxidationsmittel zugesetzt wird;

   (b) mindestens ein Teil des Desoxidationsmittels mit gelöstem Sauerstoff in dem Bad umgesetzt wird, uni Desoxidationsmitteloxid (e) zu bilden, das (die) seinerseits (ihrerseits) eine Lösung mit dem (den) Metalloxid(en) bildet (bilden); und

   (c) das Metallschmelzbad durch unter der Badoberfläche erfolgendes Einblasen von Gas in das Bad für eine Zeitspanne von mindestens gleich 9 t vor dem Einsatz der Sauerstoffsonde gerührt wird, wobei

   $t = 800\ E^{-0.4}$, und
   $E = [7250 + 9850\ \ln (1 + 0{,}676\ h)]\ V/W$

   wobei t in Sekunden angegeben ist, h die Höhe der Badoberfläche über der Ruhrgas-Einblasstelle in Metern ist, V die Rührgas-Durchflußmenge in Normalkubikmetern pro Minute ist und W das Gewicht des Bades in metrischen Tonnen ist,

   $(E = [226 + 307\ \ln (1 + 0{,}206\ h)]\ V/W$,

   wobei t in Sekunden angegeben ist, h die Höhe der Badoberflache über der Rührgas−Einblasstelle in Fuß ist, V die Rührgas−Durchflußmenge in Normalkubikfuß pro Minute ist und W das Gewicht des Bades in short tons ist.)

2. Verfahren nach Anspruch 1, wobei das Desoxidationsmittel Aluminium ist.

3. Verfahren nach Anspruch 1, wobei das Desoxidationsmittel Silizium ist.

4. Verfahren nach Anspruch 1, wobei das Desoxidationsmittel sowohl Aluminium als auch Silizium ist.

5. Verfahren nach Anspruch 1, wobei das geschmolzene Metall Stahl ist.

6. Verfahren nach Anspruch 1, wobei das geschmolzene Metall eine Nickelbasislegierung ist.

7. Verfahren nach Anspruch 1, wobei das Rührgas Argon ist.

8. Verfahren nach Anspruch 1, wobei das Rührgas Stickstoff ist.

9. Verfahren nach Anspruch 1, wobei das Desoxidationsmittel diskontinuierlich zugegeben wird.

10. Verfahren nach Anspruch 1, wobei der Desoxidationsmittelzusatz in Form eines Pulvers erfolgt, das in die Schmelze mit Gas eingeblasen wird.

11. Verfahren nach Anspruch 1, wobei der Desoxidationsmittelzusatz in Form eines Drahtes erfolgt, der kontinuierlich in das Bad eingebracht wird.

12. Verfahren nach Anspruch 1, wobei das Desoxidationsmittel aus der Titan, Vanadium, Cal−cium, Zirkonium und Seltenerdmetalle beste−henden Gruppe ausgewählt wird.

13. Verfahren nach Anspruch 5, wobei die Schmelze durch den Argon−Sauerstoff−Entkohlungsprozeß (AOD−Verfahren) raffiniert wird.

14. Verfahren nach Anspruch 13, wobei die Rührdauer mindestens 3 Minuten beträgt.

## Revendications

1. Procédé pour déterminer la concentration d'un désoxydant dans un bain de métal fondu en utilisant l'activité d'oxygène dans le bain, déterminée au moyen d'une sonde de dosage d'oxygène, comprenant les étapes consistant :

    (a) à effectuer une addition d'un désoxydant à un bain de métal fondu, contenant de l'oxygène et un ou plusieurs oxydes métalliques dissous ;

    (b) à faire réagir au moins une partie du désoxydant avec l'oxygène dissous présent dans le bain pour former un ou plusieurs oxydes de désoxydant qui forment à leur tour une solution avec le ou les oxydes métalliques ; et

    (c) à agiter le bain de métal fondu par injection d'un gaz sous la surface du bain pendant un temps au moins égal à 9t avant utilisation de la sonde de dosage d'oxygè−ne, procédé dans lequel

    $t = 800\ E^{-0.4}$, et
    $E = [7250 + 9850\ \ln(1 + 0{,}676\ h)[\ V/W$

    t étant exprimé en secondes, h désignant la hauteur, en mètres, de la surface du bain au−dessus du point d'injection de gaz d'agitation, V désignant le débit du gaz d'agitation en mètres cubes par minute en conditions normales et W désignant le poids du bain en tonnes métriques

    $(E = [226 + 307\ \ln(1 + 0{,}206\ h)]\ V/W$

    t étant exprimé en secondes, h désignant la hauteur, en feet, de la surface du bain au−dessus du point d'injection de gaz d'agitation, V désignant le débit du gaz d'agitation en $ft^3$ par minute en conditions normales, et W désignant le poids du bain en tonnes courtes).

2. Procédé suivant la revendication 1, dans lequel le désoxydant est l'aluminium.

3. Procédé suivant la revendication 1, dans lequel le désoxydant est le silicium.

4. Procédé suivant la revendication 1, dans lequel le désoxydant consiste à la fois en aluminium et en silicium.

5. Procédé suivant la revendication 1, dans lequel le métal fondu est l'acier.

**6.** Procédé suivant la revendication 1, dans lequel le métal fondu est un alliage à base de nickel.

**7.** Procédé suivant la revendication 1, dans lequel le gaz d'agitation est l'argon.

**8.** Procédé suivant la revendication 1, dans lequel le gaz d'agitation est l'azote.

**9.** Procédé suivant la revendication 1, dans lequel le désoxydant est ajouté par addition discon‐tinue.

**10.** Procédé suivant la revendication 1, dans lequel l'addition de désoxydant est effectuée par in‐sufflation de poudre dans la masse fondue au moyen d'un gaz.

**11.** Procédé suivant la revendication 1, dans lequel l'addition de désoxydant est effectuée par ad‐dition d'un fil métallique qui est introduit en continu dans la masse fondue.

**12.** Procédé suivant la revendication 1, dans lequel le désoxydant est choisi dans le groupe com‐prenant le titane, le vanadium, le calcium, le zirconium et les métaux faisant partie des ter‐res rares.

**13.** Procédé suivant la revendication 5, dans lequel la masse fondue est affinée par le procédé de décarburation à l'argon‐oxygène (procédé AOD).

**14.** Procédé suivant la revendication 13, dans le‐quel le temps d'agitation est égal à au moins 3 minutes.

F I G.  I